# EUROPEAN PATENT SPECIFICATION

(11) **EP 1 040 144 B1**
(45) Date of publication and mention of the grant of the patent: **12.03.2003**
(21) Application number: 98948527.1
(22) Date of filing: 22.09.1998
(51) Int. Cl.: C08F 222/04, C08F 222/06, C08F 216/12, C08F 210/10, C08L 35/08, C08K 5/09

(54) **SOLVENT-FREE PROCESS FOR MAKING HIGH MOLECULAR WEIGHT TERPOLYMERS OF MALEIC ANHYDRIDE, C1-C4 ALKYL VINYL ETHER AND ISOBUTYLENE, AND DENTURE ADHESIVE AND DENTIFRICE COMPOSITIONS CONTAINING SUCH TERPOLYMERS**
LÖSEMITTELFREIES VERFAHREN ZUR HERSTELLUNG VON TERPOLYMEREN VON MALEINSÄUREANHYDRID, C1-C4-ALKYL VINYLETHER UND ISOBUTYLEN MIT HOHEM MOLEKULARGEWICHT UND ZAHNPROTHESEHAFTMITTEL UND ZAHNPASTEN DIE DIESE TERPOLYMERE ENTHALTEN
PROCEDE DE FABRICATION SANS SOLVANT DE TERPOLYMERES A POIDS MOLECULAIRE ELEVE D'ANHYDRE MALIQUE, D'ETHER VINYLIQUE D'ALKYLE C1-C4 ET D'ISOBUTYLENE, COMPOSITIONS DENTIFRICES ET DE CIMENT POUR PROTHESE DENTAIRE CONTENANT CES TERPOLYMERES

(30) Priority: 02.10.1997 US 942830; 16.06.1998 US 97728; 16.06.1998 US 97895
(43) Date of publication of application: 04.10.2000
(73) Proprietor: ISP INVESTMENTS INC., Wilmington, Delaware 19801 (US)
(72) Inventor: PLOCHOCKA, Krystyna, Scotch Plains, NJ 07076 (US); PROSISE, William, E., Ramsey, NJ 07446 (US); ZHANG, Huixiang, Wayne, NJ 07470 (US)
(74) Representative: Harrison, David Christopher
(86) International application number: US9820037
(87) International publication number: WO99018140

(56) References cited:
- GB-A- 1 028 231
- US-A- 5 037 924

## Description

### BACKGROUND OF THE INVENTION

### 1. Field of the Invention

This invention relates to a solvent-free process for making high molecular weight terpolymers of maleic anhydride (MAN), C₁-C₄ alkyl vinyl ethers (AVE), and isobutylene (IB), and to dentifrice and denture adhesive compositions containing such solvent-free, high molecular weight terpolymers.

### 2. Detailed Description of the Prior Art

U.S. Pat. 5,037,924 described the preparation of terpolymers of maleic anhydride, a C₁-C₄ alkyl vinyl ether and isobutylene, for use as denture adhesives in the form of their mixed salts. These terpolymers were made by precipitation polymerization in the presence of an added solvent, for example, a cosolvent system of ethyl acetate and cyclohexane. The resultant terpolymer had only a low molecular weight of about 30,000 to 400,000. While their performance as denture adhesives was relatively good as compared to others known in the art, the terpolymers and their salts contained undesirable trace amounts of ethyl acetate and cyclohexane.

U.S. Pat. 5,334,375 described dentifrice compositions containing crosslinked copolymers of MAN, an AVE and IB, for controlling plaque.

Accordingly, it is an object of this invention to provide a solvent-free process of making high molecular weight terpolymers of MAN, an AVE and IB.

A specific object herein is to provide such solvent-free terpolymers with molecular weights of at least about 1,500,000, which, in the form of their mixed salts, perform in an excellent manner as denture adhesives, and in dentifrice compositions.

### SUMMARY OF THE INVENTION

What is described herein is a solvent-free process for making fine powders of high molecular weight alternating terpolymers of maleic anhydride (MAN), a C₁-C₄ alkyl vinyl ether (AVE) and isobutylene (IB), having a molecular structure of (A-B)ₙ, where A = MAN and B = AVE or IB, and, preferably, containing about 5 to 45 mole % of isobutylene.

The NMR spectra of these terpolymers show an alternating molecular structure (A-B)ₙ, where n is such that the terpolymer has a weight average molecular weight (GPC, water, pH 9) in excess of about 1,500,000, and a specific viscosity which is ≥ 6 (1% in DMF, 25°C).

The solvent-free process of the invention is carried out by charging the alkyl vinyl ether and isobutylene into a reactor at a mole ratio of isobutylene to alkyl vinyl ether which is substantially greater than that desired in the terpolymer, adding a radical initiator, heating the mixture to a reaction temperature of about 50 to 100°C, and feeding molten maleic anhydride over time into the reactor, wherein the mole ratio of maleic anhydride to the total charge of alkyl vinyl ether and isobutylene is not higher than 1:3. The resulting terpolymers obtained herein are odorless and free of the trace amounts of solvents characteristic of other known processes for making such terpolymers.

These terpolymers are used advantageously in denture adhesive and dentifrice compositions.

### DETAILED DESCRIPTION OF THE INVENTION

In accordance with the invention, there is provided a solvent-free process for making high molecular weight terpolymers of maleic anhydride, a C₁-C₄ alkyl vinyl ether, preferably methyl vinyl ether, and isobutylene, having an alternating molecular structure (A-B)ₙ, where A = MAN and B = AVE and/or IB. The terpolymers have a molar ratio of maleic anhydride to alkyl vinyl ether to isobutylene of about 0.50:0.45-0.25:0.05-0.45, respectively. Preferably the terpolymer includes about 5-45 mole % of IB. A most preferred composition has about 10-15% IB, as defined above. The molecular weight is at least 1,500,000 (GPC, water, pH 9), and the specific viscosity (SV) is ≥ 6 (1% in DMF, 25°C).

The terpolymers are obtained as solvent-free, fine white powders.

The mixed salt of the terpolymer, e.g. a Ca/Na or Ca/Zn mixed salt, exhibits excellent performance properties when used as a denture adhesive.

The uncrosslinked terpolymer is particularly useful as part of a dentifrice composition for controlling plaque.

A feature of the process of the invention is a solvent-free polymerization process which is carried out using an excess of alkyl vinyl ether and isobutylene as the reaction medium. Accordingly, the polymerization reaction is carried out by precharging an AVE/IB mixture of predetermined composition into a reactor, adding a radical initiator, heating to a reaction temperature of about 50° to 100°C, and feeding molten maleic anhydride into the reactor over time.

The mole ratio of MAN to the total of MVE + IB is not higher than 1:3, preferably less than 1:5. Furthermore, the mole ratio of IB to MVE in the precharge is made significantly higher than that desired in the terpolymer, preferably about 1:1 IB:MVE for a terpolymer containing 1:2 IB:MVE and about 1:2 IB:MVE for a terpolymer containing 1:4 IB:MVE.

Generally, an additional amount of MVE is added near the end of the polymerization in order to completely use up the MAN reactant.

After stripping the remaining excess MVE and IB, the terpolymer product is obtained as a fine odorless powder, without any solvent, i.e. it is solvent-free.

The invention will now be described with reference to the following examples.

### EXAMPLE 1

A 1-liter Parr stainless steel reactor equipped with an agitator, heating mantle and syringe pumps for charging reagents was sparged with nitrogen and charged with 175 g (3.00 mole) of methyl vinyl ether (MVE), 175 g (3.12 mole) of isobutylene (IB) and 0.10 g of lauroyl peroxide. The mole ratio of MVE to IB was 0.96. The charged reactor was heated to 63°C and the temperature was maintained while molten maleic anhydride (MAN)in an amount of 39.2 g (0.400 mole) was fed into the reactor over a period of 2 hours. The mole ratio of MVE and IB to MAN was 15.3. After holding for 3 hours at 65°C, an additional amount of 50 g (0.86 mole) MVE was added. Then the temperature was raised to 70°C and maintained for 1 hour. Thereafter the reactor was cooled to room temperature, the pressure was released and the reactor discharged. The reaction product was recovered as a fine, white powder which was dried for 1 hour in a vacuum oven at 65°C. The product was a uniform, fine white powder (62 g), without any unreacted MAN. The specific viscosity (SV), as measured in a 1% DMF solution at 25°C was 6.94. A ¹³C NMR spectrum showed that the mole ratio of monomers in the terpolymer of MAN:MVE:IB was 0.50:0.34:0.16. The weight average molecular weight (GPC, water, pH 9) of the powder was 2,170,000.

### EXAMPLES 2-5

The procedure of Example 1 was followed to provide terpolymers having different ratios of MAN:MVE:IB. The results are shown in Table 1 below.

**TABLE 1**

| | Example No. | | | |
|---|---|---|---|---|
| Polymer | 2 | 3 | 4 | 5 |
| MVE, g (mol) | 100 (1.72) | 200 (344) | 167 (2.87) | 200 (3.44) |
| | | | | |
| IB, g (mol) | 150 (2.67) | 50 (0.89) | 83 (1.49) | 25 (0.44) |
| | | | | |
| MAN, g (mol) | 41 (0.418) | 41 (0.418) | 41 (0.418) | 41 (0.418) |
| | | | | |
| MVE/IB ratio in the reaction (by mole) | 0.39:0.61 | 0.79:0.11 | 0.66:0.34 | 0.89:0.11 |
| | | | | |
| MAN:MVE:IB ratio in polymer (by ¹³C NMR, in mole fractions) | 0.50:0.28:0.21 | 0.50:0.46:0.07 | 0.50:0.40:0.10 | 0.50:0.44:0.05 |
| | | | | |
| SV (1%, DMF) | 7.71 | 11.11 | 6.96 | 14.46 |
| | | | | |
| Mw (GPC, water, pH 9) | 2,100,000 | 2,101,000 | 2,180,000 | 2,150,000 |

### EXAMPLE 6

### DENTURE ADHESIVE COMPOSITIONS

The terpolymers of Examples 1-5 were converted to their Ca/Na (7/2 by equiv.) and Ca/Zn (3/1 by equiv.) mixed salts, at 70% neutralization of carboxyl groups. The products were compared to equivalent reference mixed salts made from Gantrez® AN 169 BF, i.e. to a commercially available MAN-MVE (1:1) copolymer, commonly used as a starting material for denture adhesives.

The reference Gantrez® AN 169 BF material was selected to have a molecular weight of 2,450,000 (GPC) which was higher than any of the invention terpolymers in Examples 1 through 5 herein. Therefore, any advantage in performance of the invention terpolymers can be attributed to their molecular structure, rather than to their particular molecular weight. The terpolymer salts of the invention containing 5 to 45% IB, preferably 10-25% IB, exhibited a significantly longer and stronger hold property than the reference material based on results of standard denture adhesive testing, according to the procedure described in detail in U.S. Pat. 5,037,924.

In contrast, a MAN-MVE-IB copolymer with monomer ratios of 0.50:0.37:0.13 and a molecular weight of 223,000, made according to U.S. Pat. 5,037,924, displayed significantly poorer performance in denture adhesives than a Gantrez® AN 169 BF reference material. Similarly, high molecular weight MAN-MVE-IB terpolymers having less than about 5% or more than about 45% IB did not show any significant improvement in performance as a denture adhesive as compared to the Gantrez® AN 169 reference.

### EXAMPLE 7

### TEST METHOD FOR DENTIFRICE COMPOSITIONS

An in vitro test was used to evaluate the performance of a given polymer as a dentifrice. The test measured the triclosan* (2,4,4'-trichloro-2'-hydroxy-diphenyl ether) retention and uptake after the polymer was applied to the surface of hydroxy apatite (HAP), an artificial tooth surface. This test is correlative with actual brushing with toothpaste followed by rinsing with water.
* Triclosan is an antibacterial commonly used to reduce plaque formation

HAP is a mineral phase of teeth which can provide a uniform surface size, and allow for quantitative and reproducible results. The HAP material was prepared in the form of a disc as per reference by placing in a die having a diameter of 13 mm and compressing. Then the resultant pellet was dried and finally sintered at 800°C for 4 hours.

Uptake of triclosan was measured by incubating the HAP disc in artificial saliva overnight at 37°C and immersing it in a sample dentifrice solution containing a given polymer and triclosan at 37°C for 30 minutes with continuous shaking. The disc then was rinsed in distilled water and dried in a stream of air. The thus-treated disc was placed in acetonitrile solvent to extract triclosan from the disc. The amount of triclosan present in the solvent was then determined by HPLC.

Retention of triclosan on the saliva-coated HAP disc was measured by treating the disc with the dentifrice solution, rinsing the disc with water, and reincubating in artificial saliva for 60 minutes with continuous shaking. The disc was then removed from the saliva, rinsed with water and placed in acetonitrile. The amount of triclosan retained on the disc was measured by HPLC. The control sample did not contain any polymer.

**TABLE 2**

| Compositions of Liquid Phase Dentifrice Solution for Testing | |
|---|---|
| Ingredient | Wt % |
| Terpolymer of Invention | 0-2.0 |
| Triclosan | 0.30 |
| Sodium lauaryl sulfate | 2.46 |
| Sorbitol | 20.00 |
| Propylene glycol | 13.00 |
| Sodium fluoride | 0.24 |
| Water | 61-63 |

The results are given in Table 3 as follows:

**TABLE 3**

| Terpolymer in Example No. | % Polymer | Triclosan Uptake/Retention micrograms/Disc | |
|---|---|---|---|
| | | Uptake | Retention |
| 1 (Control) | - | 75 | 45 |
| 2 | 2 | 160 | 100 |
| 3 | 0.6 | 115 | 70 |

The results herein demonstrate the effectiveness of the new polymer in dentifrice compositions as compared to others previously used in the art.

## Claims

1. A solvent-free process for making solvent-free, fine powders of high molecular weight alternating terpolymers of maleic anhydride, a C₁-C₄ alkyl vinyl ether and isobutylene having molecular structure (A-B)ₙ, where A is maleic anhydride and B is alkyl vinyl ether or isobutylene and containing about 5 to 45 molar % of isobutylene, which have a weight average molecular weight (GPC, water, pH 9) in excess of about 1,500,000, and a specific viscosity ≥ 6 (1% in DMF), which comprises charging the alkyl vinyl ether and isobutylene into a reactor, the mole ratio of isobutylene to alkyl vinyl ether being substantially greater than that desired in the terpolymer, adding a radical initiator, heating the mixture to a reaction temperature of about 50 to 100°C, feeding molten maleic anhydride into the thus charged reactor over time, the mole ratio of maleic anhydride fed therein to the total charge of alkyl vinyl ether and isobutylene being no higher than 1:3, optionally adding an additional amount of alkyl vinyl ether to complete the reaction and later stripping excess isobutylene and alkyl vinyl ether after polymerization is completed.

2. A process according to claim 1 wherein said C₁-C₄ alkyl vinyl ether is methyl vinyl ether.

3. A process according to claim 1 wherein the mole ratio of maleic anhydride to alkyl vinyl ether to isobutylene in the terpolymer is 0.50:0.45-0.25:0.05-0.45, respectively.

4. A process according to claim 1 wherein the mole ratio of isobutylene to alkyl vinyl ether in the charge is about 1:1 for a terpolymer with about a 1:2 ratio of said monomers.

5. Solvent-free, fine white powders of an alternating terpolymer of maleic anhydride, a C₁-C₄ alkyl vinyl ether and isobutylene having molecular structure (A-B)ₙ, where A is maleic anhydride and B is alkyl vinyl ether or isobutylene, and having a mole ratio of maleic anhydride to alkyl vinyl ether to isobutylene of 0.50:0.45-0.25:0.05-0.45, and about 5 to 25 mole % of isobutylene, which has a weight average molecular weight of at least about 1,500,000, as measured by GPC in water (pH 9), and a specific viscosity of ≥ 6, as measured on 1% solutions in DMF.

6. A denture adhesive composition including the solvent-free, high molecular weight terpolymer of claim 5 in the form of its mixed salt, the two cations being selected from Na⁺, Ca⁺⁺, Zn⁺⁺, Al⁺⁺⁺ and Mg⁺⁺, preferably the Ca⁺⁺/Na⁺ or Ca⁺⁺/Zn⁺⁺ mixed salt.

7. A dentifrice composition including an antibacterial enhancing amount of the solvent-free, high molecular weight uncrosslinked terpolymer of claim 5.

## Patentansprüche

1. Lösungsmittelfreies Verfahren zur Herstellung lösungsmittelfreier, feiner Pulver aus hochmolekularen alternierenden Terpolymeren aus Maleinsäureanhydrid, einem C₁₋₄-Alkylvinylether und Isobutylen mit der Molekülstruktur (A-B)ₙ, worin A Maleinsäureanhydrid ist und B Alkylvinylether oder Isobutylen ist, die etwa 5 bis 45 Mol-% Isobutylen enthalten, die ein gewichtsmittleres Molekulargewicht (GPC, Wasser, pH 9) von über etwa 1.500.000 und eine spezifische Viskosität ≥ 6 (1 % in DMF) aufweisen, umfassend das Einfüllen des Alkylvinylethers und Isobutylens in einen Reaktor, wobei das Molverhältnis zwischen Isobutylen und Alkylvinylether deutlich höher ist als das im Terpolymer gewünschte, die Zugabe eines Radikalinitiators, das Erhitzen des Gemischs auf eine Reaktionstemperatur von etwa 50 bis 100 °C, die allmähliche Zufuhr von geschmolzenem Maleinsäureanhydrid in den so befüllten Reaktor, wobei das Molverhältnis zwischen dem zugeführten Maleinsäureanhydrid und der Gesamtladung an Alkylvinylether und Isobutylen nicht über 1:3 liegt, die optionale Zugabe einer zusätzlichen Menge an Alkylvinylether, um die Reaktion vollständig ablaufen zu lassen, und das spätere Abstreifen von überschüssigem Isobutylen und Alkylvinylether, nachdem die Polymerisation vollständig abgelaufen ist.

2. Verfahren nach Anspruch 1, worin der C₁₋₄-Alkylvinylether Methylvinylether ist.

3. Verfahren nach Anspruch 1, worin das Molverhältnis zwischen Maleinsäureanhydrid, Alkylvinylether und Isobutylen im Terpolymer jeweils 0,50 : 0,45-0,25 : 0,05-0,45 beträgt.

4. Verfahren nach Anspruch 1, worin das Molverhältnis zwischen Isobutylen und Alkylvinylether in der Ladung bei einem Terpolymer mit etwa einem 1:2-Verhältnis der Monomere etwa 1:1 beträgt.

5. Lösungsmittelfreie, feine weiße Pulver aus einem alternierenden Terpolymer aus Maleinsäureanhydrid, einem C₁₋₄-Alkylvinylether und Isobutylen mit der Molekülstruktur (A-B)ₙ, worin A Maleinsäureanhydrid ist und B Alkylvinylether oder Isobutylen ist, das ein Molverhältnis zwischen Maleinsäureanhydrid, Alkylvinylether und Isobutylen von 0,50 : 0,45-0/25 : 0,05-0,45 aufweist, etwa 5 bis 25 Mol-% Isobutylen enthält und ein mittels GPC in Wasser (pH 9) gemessenenes gewichtsmittleres Molekulargewicht von zumindest etwa 1.500.000 sowie eine in 1%igen DMF-Lösungen gemessene spezifische Viskosität ≥ 6 aufweist.

6. Zahnprothesenkleberzusammensetzung, die das lösungsmittelfreie, hochmolekulare Terpolymer nach Anspruch 5 in Form eines Mischsalzes davon, bei dem die beiden Kationen aus Na⁺, Ca⁺⁺, Zn⁺⁺, Al⁺⁺⁺ und Mg⁺⁺ ausgewählt sind, vorzugsweise als Ca⁺⁺/Na⁺-oder Ca⁺⁺/Zn⁺⁺-Mischsalz enthält.

7. Zahnpastenzusammensetzung, die eine die antibakterielle Wirkung verstärkende Menge eines lösungsmittelfreien, hochmolekularen, unvernetzten Terpolymers nach Anspruch 5 umfasst.

## Revendications

1. Procédé sans solvant pour produire des poudres fines, sans solvant, de terpolymères alternés de fort poids moléculaire d'anhydride maléique, d'un alkyl C₁-C₄ vinyl éther et d'isobutylène ayant une structure moléculaire (A-B)ₙ où A est l'anhydride maléique et B est l'alkyl vinyl éther ou l'isobutylène et contenant environ 5 à 45% en moles d'isobutylène, qui ont un poids moléculaire moyen en poids (GPC, eau, pH 9) en excès d'environ 1 500 000 et une viscosité spécifique ≥ 6 (1% dans DMF), qui consiste à charger l'alkyl vinyl éther et l'isobutylène dans un réacteur, le rapport molaire de l'isobutylène à l'alkyl vinyl éther étant sensiblement plus grand que celui souhaité dans le terpolymère, à ajouter un initiateur de radicaux, à chauffer le mélange jusqu'à une température de réaction d'environ 50 à 100°C, à fournir l'anhydride maléique fondu dans le réacteur ainsi chargé avec le temps, le rapport molaire de l'anhydride maléique fourni à la charge totale d'alkyl vinyl éther et d'isobutylène ne dépassant pas 1:3, à ajouter facultativement une quantité additionnelle d'alkyl vinyl éther pour terminer la réaction et à extraire ultérieurement l'excès d'isobutylène et d'alkyl vinyl éther après avoir terminé la polymérisation.

2. Procédé selon la revendication 1 où ledit alkyl C₁-C₄ vinyl éther est méthyl vinyl éther.

3. Procédé selon la revendication 1 où le rapport molaire de l'anhydride maléique à l'alkyl vinyl éther à l'isobutylène dans le terpolymère est de 0,50:0,45-0,25:0,05-0,45, respectivement.

4. Procédé selon la revendication 1 où le rapport molaire de l'isobutylène à l'alkyl vinyl éther dans la charge est d'environ 1:1 pour un terpolymère avec à peu près un rapport de 1:2 desdits monomères.

5. Poudres fines, blanches, sans solvant d'un terpolymère alterné d'anhydride maléique, d'un alkyl C₁-C₄ vinyl éther et d'isobutylène ayant la structure moléculaire (A-B)ₙ, où A est l'anhydride maléique et B est un alkyl vinyl éther ou de l'isobutylène et ayant un rapport molaire de l'anhydride maléique à l'alkyl vinyl éther à l'isobutylène de 0,50:0,45-0,25:0,05-0,45 et environ 5 à 25% en moles d'isobutylène, qui a un poids moléculaire moyen en poids d'au moins environ 1 500 000, en mesurant par GPC dans l'eau (pH 9), et une viscosité spécifique ≥ 6, en mesurant sur une solution à 1% dans DMF.

6. Composition adhésive pour prothèse dentaire comprenant le terpolymère de poids moléculaire élevé, sans solvant, de la revendication 5 sous la forme de son sel mélangé, les deux cations étant sélectionnés parmi Na⁺, Ca⁺⁺, Zn⁺⁺, Al⁺⁺⁺ et Mg⁺⁺, de préférence le sel mélangé de Ca⁺⁺/Na⁺⁺ ou Ca⁺⁺/Zn⁺⁺.

7. Composition dentifrice comprenant une quantité d'activation antibactérienne du terpolymère non réticulé sans solvant de poids moléculaire élevé de la revendication 5.
